# EUROPEAN PATENT SPECIFICATION

(11) **EP 4 133 967 B1**
(45) Date of publication and mention of the grant of the patent: **25.03.2026**
(21) Application number: 20930602.6
(22) Date of filing: 11.09.2020
(51) Int. Cl.: A45D 20/12, A61H 39/04, A61N 5/06

(54) **DIFFUSER AND HAIR DRYER INCLUDING SAME**
DIFFUSOR UND HAARTROCKNER DAMIT
DIFFUSEUR ET SÈCHE-CHEVEUX LE COMPRENANT

(30) Priority: 10.04.2020 KR 20200044035
(43) Date of publication of application: 15.02.2023
(73) Proprietor: LG Electronics, Inc., Yeongdeungpo-gu Seoul 07336 (KR)
(72) Inventor: KIM, Kyoungtae, Seoul 08592 (KR); SHIN, Daekee, Seoul 08592 (KR); PARK, Sunjung, Seoul 08592 (KR); KU, Yunhee, Seoul 08592 (KR); KIM, Dongwon, Seoul 08592 (KR)
(74) Representative: Vossius & Partner Patentanwälte Rechtsanwälte mbB
(86) International application number: PCT/KR2020/012288
(87) International publication number: WO 2021/206233

(56) References cited:
- BR-A2- 102018 010 209
- JP-A- 2011 019 631
- JP-A- 2019 118 729
- JP-A- 2019 180 473
- JP-B1- 6 346 700
- JP-U- S5 579 904
- KR-A- 20100 066 043
- KR-A- 20180 017 193
- KR-A- 20190 072 169
- KR-A- 20190 084 801
- KR-A- 20200 033 115
- KR-Y1- 850 002 139
- US-A- 5 715 847
- US-A1- 2011 209 721

## Description

### [Technical Field]

The present disclosure relates to a diffuser and a hair dryer including the same, more particularly, to a diffuser coupled to a main body of a hair dryer to provide gas to a user and the hair dryer including the same.

### [Background Art]

When removing moisture from wet hair of a human body as much as desired or when styling the hair from a current shape to a desired shape, a hair dryer that discharges gas through a gas outlet may be used.

In one example, the hair dryer may provide gas characteristics desired by a user, such as a gas temperature, a gas speed, a gas flow area, and the like through a diffuser. The diffuser may be coupled to a main body of the hair dryer to change the gas characteristics and provide the changed gas characteristics to the user. Further, the diffuser may include care means such as a massage protrusion to manage a scalp health and the like of the user.

In connection, Korean Utility Model Application Publication No. 20-2011-0002484 discloses a diffuser disposed in a hair dryer. The diffuser disclosed in the above document discloses a massage protrusion that may perform user's scalp and hair care.

As described above, the diffuser may provide a massage effect or gas to the user in a state of being adjacent to the scalp and the hair of the user by including the massage protrusion and the like.

However, a head of the human may be understood as a curved face whose surface has a curvature. Therefore, in order to provide the effective care effect to the user, designing the diffuser in consideration of an efficient shape, which is in consideration of a user's head, becomes an important task in this technical field.

US 5 715 847 A discloses a hair styling implement adapted to be attached to an air outlet opening of a hair dryer and equipped with a housing and a plurality of hair pickup elements and an adjusting element that is movable relative to the housing.

US 2011/209721 discloses the preamble of claim 1.

### [Disclosure]

### [Technical Problem]

Embodiments of the present disclosure are intended to provide a diffuser having an efficient shape corresponding to a user's head and a hair dryer including the same.

In addition, embodiments of the present disclosure are intended to provide a diffuser and a hair dryer including the same capable of effectively improving a user's scalp and hair care effect.

### [Technical Solution]

The present invention is defined by independent claims 1 and 8, the dependent claims describe embodiments of the present invention.

The present invention provides a diffuser having a shape capable of effectively accessing user's scalp and hair and providing a care effect, and a hair dryer including the same.

In a human body, a surface of a head corresponds to a curved surface having a curvature, not a plane. Thus, when a front end of the diffuser that may provide gas or the care effect to a user has an usual plain round structure, a distance between a portion of the diffuser and the user's head may be different from a distance between a remaining portion of the diffuser and the user's head.

For example, the remaining portion of the diffuser may be spaced apart from the user's head even when the portion of the diffuser is in close contact with the user's head. Gas may leak through a region between the remaining portion of the diffuser and the user's head, a massage protrusion may become away from the scalp of the user, or light irradiated for the care effect may leak outside.

That is, when the diffuser has a general circular structure that does not take the user's head into consideration, a light amount of an LED module for scalp care may be lost and the gas provided to the user may leak outside.

Accordingly, an embodiment of the present disclosure is to minimize loss of the LED light amount and air volume and effectively increase ease of use by applying a triangular shape structure to a diffusing case.

The diffusing case may include three cut regions obtained by cutting a cone to form 120 degrees with a center of an axis. Each cut region may have a shape allowing an entirety of the cut region to be in close contact with a substantially spherical surface.

The diffuser according to an embodiment of the present disclosure as described above includes a diffusing case having a rear side removably coupled to a main body of a hair dryer, wherein gas discharged from the main body is introduced into the diffusing case through a gas inlet hole defined at the rear side, wherein an open surface is defined at a front side of the diffusing case such that the gas introduced into the diffusing case is discharged to outside through the open surface.

The diffusing case includes a front circumferential portion surrounding the open surface, the front circumferential portion includes a first portion and a second portion, and the first portion is at least partially located forwardly of the second portion.

The first portion includes a plurality of first portions and the second portion includes a plurality of second portions, and the plurality of first portions and the plurality of second portions are alternately arranged with each other along a circumference of the open surface.

In the front circumferential portion, the first portion and the second portion extend along the circumference of the open surface while respectively having curvatures when viewed from the side.

The second portion may have a concave curved shape in a rearward direction, and the distance from the front end of the second portion to the rear side may increase in a direction away from a center of the second portion.

The first portion may have a convex curved shape in a forward direction, and the distance from the front end of the first portion to the rear side may increase in a direction toward a center of the first portion.

The diffuser may further include a light irradiator disposed inside the diffusing case and irradiating light forwardly of the diffusing case through the open surface.

The diffuser further includes a discharge cover coupled to the front side of the diffusing case to shield the open surface, wherein the discharge cover includes a gas discharge hole defined therein for discharging the gas inside the diffusing case to the outside.

The discharge cover may include a massage protrusion protruding forward to press a target in front of the discharge cover.

The discharge cover may include an edge having a curvature corresponding to the front circumferential portion when viewed from the side, wherein the edge is located inward of the front circumferential portion.

The discharge cover has a front surface directed in a forward direction having a shape of being indented rearwards in a direction toward a center of the front surface.

In one example, a hair dryer according to an embodiment of the present disclosure includes a main body including a gas outlet for discharging gas therethrough, a handle extending from the main body, and a diffuser removably coupled to the main body to introduce the gas discharged from the gas outlet therein and discharge the gas introduced therein to outside.

The diffuser includes a diffusing case having a rear side removably coupled to the main body of the hair dryer, wherein gas discharged from the gas outlet is introduced into the diffusing case through a gas inlet hole defined at the rear side, wherein an open surface is defined at a front side of the diffusing case such that the gas introduced into the diffusing case is discharged to outside through the open surface.

The diffusing case includes a front circumferential portion surrounding the open surface, the front circumferential portion includes a first portion and a second portion, and the first portion is at least partially located forwardly of the second portion.

In the front circumferential portion, the first portion and the second portion extend along the circumference of the open surface while respectively having curvatures when viewed from the side.

The second portion may have a concave curved shape in a rearward direction, and the distance from the front end of the second portion to the rear side may increase in a direction away from a center of the second portion. The first portion may have a convex curved shape in a forward direction, and the distance from the front end of the first portion to the rear side may increase in a direction toward a center of the first portion.

### [Advantageous Effects]

Embodiments of the present disclosure provides the diffuser having the efficient shape corresponding to the user's head and the hair dryer including the same.

In addition, embodiments of the present disclosure provides the diffuser and the hair dryer including the same capable of effectively improving the user's scalp and hair care effect.

### [Description of Drawings]

FIG. 1 is a view showing a hair dryer according to an embodiment of the present disclosure;
FIG. 2 is a view showing a state in which a diffuser is separated from a hair dryer according to an embodiment of the present disclosure;
FIG. 3 is a view showing an internal cross-section of a hair dryer shown in FIG. 2;
FIG. 4 is a view showing a gas outlet in a hair dryer according to an embodiment of the present disclosure;
FIG. 5 is a view showing a diffuser according to an embodiment of the present disclosure;
FIG. 6 is a view showing an exploded view of a diffuser according to an embodiment of the present disclosure;
FIG. 7 is a view showing an internal cross-section of a diffuser according to an embodiment of the present disclosure;
FIG. 8 is a view showing a diffuser according to an embodiment of the present disclosure viewed from the front;
FIG. 9 is a view showing a first portion formed on a diffuser according to an embodiment of the present disclosure viewed from the side; and
FIG. 10 is a view showing a second portion formed on a diffuser according to an embodiment of the present disclosure viewed from the side.

### [Best Mode]

Hereinafter, an embodiment of the present disclosure will be described in detail with reference to the accompanying drawings to be easily implemented by those skilled in the art to which the present disclosure belongs.

However, the present disclosure may be implemented in many different forms and is not limited to embodiments described herein. In addition, in order to clearly describe the present disclosure, components irrelevant to the description are omitted, and like reference numerals are assigned to similar components throughout the specification.

In this specification, duplicate descriptions of the same components are omitted.

Further, in this specification, it will be understood that when a component is referred to as being "connected with" another component, the component may be directly connected with the other component or intervening components may also be present. In contrast, it will be understood that when a component is referred to as being "directly connected with" another component in this specification, there are no intervening components present.

Further, in this specification, the terminology used herein is for the purpose of describing a specific embodiment only and is not intended to be limiting of the present disclosure.

Further, in this specification, the singular forms "a" and "an" are intended to include the plural forms as well, unless the context clearly indicates otherwise.

Further, in this specification, it will be further understood that the terms "comprises", "comprising", "includes", and "including" specify the presence of the certain features, numbers, steps, operations, elements, and parts or combinations thereof, but do not preclude the presence or addition of one or more other features, numbers, steps, operations, elements, and parts or combinations thereof.

Further, in this specification, the term 'and/or' includes a combination of a plurality of listed items or one of the plurality of listed items. In this specification, 'A or B' may include 'A', 'B', or 'both A and B'.

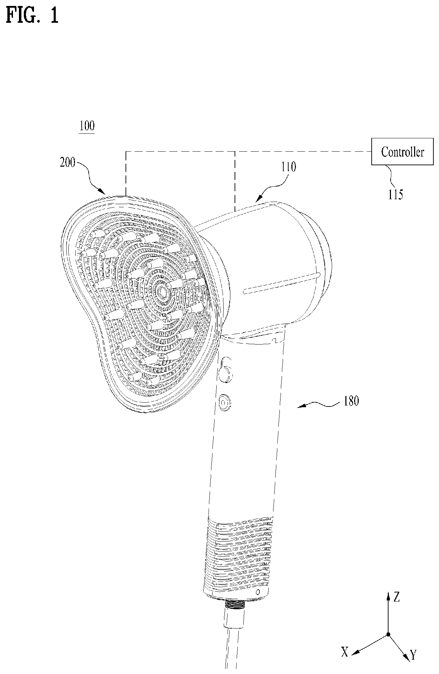

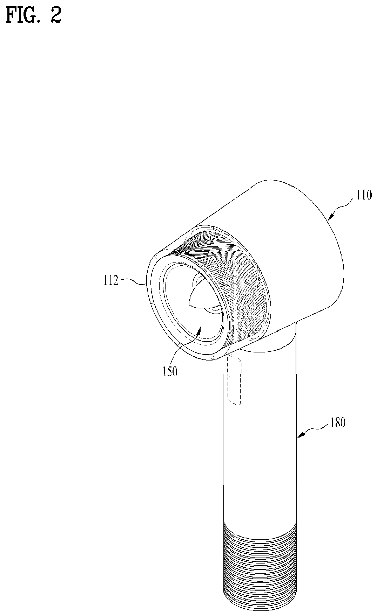

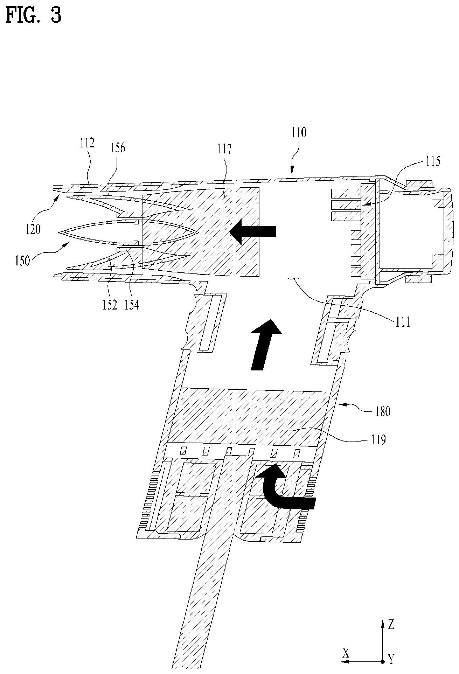

FIG. 1 is a view showing a hair dryer 100 according to an embodiment of the present disclosure. FIG. 2 is a view showing a state in which a diffuser 200 is separated from the hair dryer 100 shown in FIG. 1. Further, FIG. 3 is a view showing an internal cross-section of the hair dryer 100 shown in FIG. 2.

The hair dryer 100 according to an embodiment of the present disclosure includes a main body 110, a handle 180, and a diffuser 200 as shown in FIG. 1. In addition, as shown in FIG. 2, the main body 110 includes a gas outlet 150 through which gas introduced from outside is discharged.

As shown in FIG. 3, the main body 110 may include a gas flow path 111 through which the gas flows may be defined therein and the gas outlet 150 through which internal gas is discharged to the outside. The main body 110 may have an extended shape along a front and rear direction and may have various cross-section shapes such as circular or polygonal shapes when viewed from the front.

In the present disclosure, front, rear, left, right, top, and bottom definitions may be made centering on the main body 110. For example, referring to FIG. 2, the gas outlet 150 may be disposed at a front side of the main body 110, and the handle 180 may have a shape extending substantially downward from the main body 110.

The gas flowing inside the main body 110 may be introduced through a gas inlet, and the gas inlet may be disposed on the main body 110 or the handle 180. As shown in FIGS. 1 to 3, when the gas inlet is disposed on the handle 180, the gas flow path 111 may extend from the handle 180 to the main body 110, specifically, from the gas inlet to the gas outlet 150.

The gas may be introduced from the outside through the gas inlet disposed on the main body 110 or the handle 180, and the introduced gas may flow along the gas flow path 111 and be discharged to the outside through the gas outlet 150.

The handle 180 may extend from the main body 110. Referring to FIGS. 1 to 3, the handle 180 extending substantially downward from the main body 110 is illustrated. The handle 180 may be integrally molded with the main body 110, or separately manufactured from the main body 110 and coupled to the main body 110.

When the handle 180 is manufactured separately from the main body 110 and coupled to the main body 110, the handle 180 may be disposed such that a longitudinal direction thereof with respect to the main body 110 is fixed or variable.

For example, the handle 180 may have a hinge coupling portion, and may be coupled to the main body 110 such that the longitudinal direction of the handle 180 is changeable, that is, the handle 180 is foldable relative to the main body 110.

The handle 180 may be a portion grabbed by a hand of a user, and thus may have a shape for improving grip convenience. The extending direction of the handle 180 may vary. However, for convenience of description below, the direction in which the handle 180 extends from the main body 110 will be described as a downward direction.

Referring to FIG. 3, the hair dryer 100 according to an embodiment of the present disclosure includes a fan 119 capable of flowing the gas and adjusting a speed of the discharge gas discharged through the gas outlet 150. The fan 119 may be disposed on the gas flow path 111 to flow the gas and may be disposed inside the main body 110 or inside the handle 180.

For example, when the gas inlet is disposed on the handle 180, the gas flow path 111 may extend from the gas inlet of the handle 180 to the gas outlet 150 of the main body 110, and the fan 119 may be disposed on the gas flow path 111 located in the handle 180.

In addition, a temperature adjuster 117 that may adjust a temperature of the discharge gas may be disposed inside the main body 110. The temperature adjuster 117 may be disposed in various forms and may be disposed at various positions. In FIG. 2, the temperature adjuster 117 disposed inside the main body 110 is schematically illustrated.

In addition, the temperature adjuster 117 may be disposed in various types. The temperature adjuster 117 may be in a scheme of heating the gas by providing current to a coil-shaped resistor to generate heat.

However, the resistor of the temperature adjuster 117 may not necessarily be in the shape of the coil, and may be disposed in various types, such as a thermoelement, capable of heating the gas or adjusting the temperature of the gas.

A method for operating the hair dryer 100 according to an embodiment of the present disclosure will be schematically described with gas flow.

First, the user manipulates a power button disposed on the main body 110 or the handle 180. When the power button is turned on, the gas is introduced into the hair dryer 100 through the gas inlet as the fan 119 is operated.

The gas introduced through the gas inlet flows along the gas flow path 111 by the fan 119 toward the gas outlet 150, and the discharge gas is discharged from the gas outlet 150 to be provided to the user. In this process, a flowing speed of the gas on the gas flow path 111 may be adjusted by the fan 119 and a temperature of the gas on the gas flow path 111 may be adjusted by the temperature adjuster 117.

In one example, referring to FIGS. 1 and 2, the hair dryer 100 according to an embodiment of the present disclosure may include a controller 115. The controller 115 may be connected not only to the fan 119, the temperature adjuster 117, the power button, and a manipulator, but also to a light irradiator 260, a proximity sensor 269, a moisture measurement protrusion 312, and the like of the diffuser 200 to be described later, and control the above described components.

The controller 115 may be disposed on one of the diffuser 200, the main body 110, and the handle 180. Alternatively, the controllers 115 may be respectively arranged on all of the diffuser 200, the main body 110, and the handle 180. For example, as shown in FIG. 2, the controller 115 may be disposed on the main body 110 to be signally connected to the diffuser 200, or the plurality of controllers 115 may be respectively arranged on the diffuser 200 and the main body 110.

Adjusting operating states of the fan 119 and the temperature adjuster 117 may be performed by manipulator manipulation by the user or may be automatically performed based on an operation mode preset in the controller 115.

In addition, when a distance to a target located in front of the diffuser 200 is identified to be equal to or less than a reference distance through the proximity sensor 269 of the diffuser 200, the controller 115 may control the light irradiator 260 of the diffuser 200 to irradiate light.

In addition, the controller 115 may identify an impedance of the target located in front of the diffuser 200 through the moisture measurement protrusion 312 of the diffuser 200, and determine a moisture amount of the target through the impedance.

As the moisture amount increases, the controller 115 may control the fan 119 such that the gas speed at the gas outlet 150 increases, control the temperature adjuster 117 such that the gas temperature increases, or control the light irradiator 260 such that a light amount of the light irradiator 260 increases.

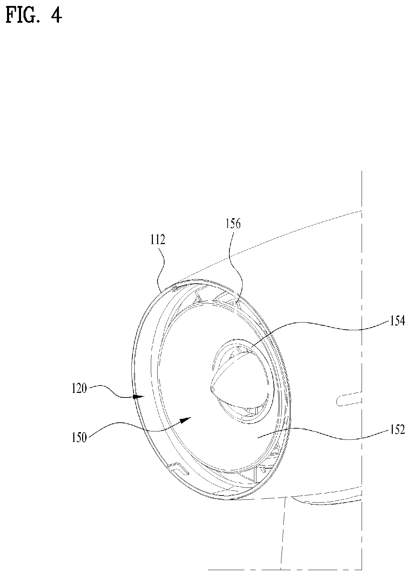

In one example, FIG. 4 shows the gas outlet 150 disposed on the main body 110. As shown in FIG. 1 or 3, the main body 110 may have a cross-section in an approximately circular shape and may have a length. However, the cross-section shape of the main body 110 may be varied as needed.

The gas outlet 150 of the hair dryer 100 according to an embodiment of the present disclosure will be described in detail with reference to FIG. 3.

At least a portion of the gas flow path 111 may be defined inside the main body 110, and one side of the main body 110 is opened. For example, the main body 110 may extend in the front and rear direction and a front surface thereof may be opened.

Opened one side of the main body 110 may be in communication with the gas flow path 111. In one example, the gas outlet 150 may be disposed on the main body 110 to shield the opened one side of the main body 110.

The opened one side of the main body 110 may correspond to an end of the gas flow path 111, and the end of the gas flow path 111 may correspond to the gas outlet 150. For example, the gas outlet 150 may be composed of the gas flow path 111 exposed through the open front surface of the main body 110 and an outer wall front end 112 of the main body 110.

In one example, as shown in FIG. 4, in an embodiment of the present disclosure, the gas outlet 150 may include a center portion 154 and side portion 156 through which the gas is discharged. The gas flowing along the gas flow path 111 may be simultaneously delivered to the center portion 154 and the side portion 156 to be discharged to the outside.

The center portion 154 and the side portion 156 may correspond to discharge holes through which the gas is discharged from the gas outlet 150. The center portion 154 may be defined at a central side on the cross-section of the gas outlet 150, and a cross-section shape thereof may be circular. However, a shape of the center portion 154 may be a polygonal shape such as a square and the like as needed, and a size of a diameter thereof may also be varied as needed.

The side portion 156 may be defined to surround the center portion 154. For example, as shown in FIG. 4, the center portion 154 may be defined in a substantially circular shape at the center of the gas outlet 150, and the side portion 156 may be an opening in a shape of a ring in which the center portion 154 is defined at a center thereof.

In the present disclosure, the ring shape may have an extended shape of a closed curve shape. For example, FIG. 4 discloses the side portion 156 having a circular ring shape.

The ring shape may not necessarily be circular, and may be, for example, a polygonal ring shape such as a triangle, a square, or the like. That is, in an embodiment of the present disclosure, the side portion 156 may be in the circular ring or the polygonal ring shape, and FIG. 4 shows the side portion 156 having a substantially circular ring shape.

Further, the center portion 154 and the side portion 156 may be in communication with the same gas flow path 111 together. Referring to FIG. 3, there is one gas flow path 111 extending from the handle 180 inside the main body 110. The center portion 154 and the side portion 156 of the gas outlet 150 are in communication with the gas flow path 111 together to discharge the gas at the same time.

The discharge gas discharged from the side portion 156 may form a sense of volume for an entirety of the discharge gas discharged through the gas outlet 150. That is, a cross-sectional area of the entirety of the discharge gas may correspond to a size of a closed cross-section formed by the side portion 156.

However, the discharge gas of the side portion 156 may be diffused while being flowed, and a portion of the gas flow may be distributed toward a center on the cross-section where the gas is not discharged by the side portion 156, and thus, the cross-sectional area of the discharge gas may be reduced.

Accordingly, in an embodiment of the present disclosure, the center portion 154 is defined at a center of the side portion 156, and the phenomenon in which the discharge gas of the side portion 156 is distributed toward the center on the cross-section is suppressed by discharge gas of the center portion 154.

That is, the discharge gas of the center portion 154 flows from the center on the cross-section of the entire discharge gas of the gas outlet 150, and suppresses the discharge gas of the side portion 156 from being distributed toward the center during the flow process, so that it may be advantageous for the entire discharge gas to maintain an initial cross-sectional area thereof.

Accordingly, discharge gas having a large cross-sectional area may be provided to the user, and the user may perform dry using the bulky gas. For example, the entire discharge gas with the volume formed through the center portion 154 and the side portion 156 may allow the user to perform the dry in a larger area.

Further, in an embodiment of the present disclosure, because the center portion 154 and the side portion 156 are in communication with one gas flow path 111, the gas flow paths 111 respectively for the center portion 154 and the side portion 156 may not separately defined. Thus, it may be advantageous in terms of design and may be efficient in providing three-dimensional discharge gas to the user.

In one example, referring to FIG. 4, in an embodiment of the present disclosure, the gas outlet 150 further includes a discharge base 152 disposed on the opened one side of the main body 110. The center portion 154 may be defined at a center of the discharge base 152, and the side portion 156 may be defined between an outer circumferential surface of the discharge base 152 and an outer wall of the main body 110.

FIG. 4 illustrates the discharge base 152 coupled to the opened one side of the main body 110. The discharge base 152 may be disposed to correspond to an opened cross-sectional shape of the one side of the main body 110, but may not be limited thereto and may be formed in various shapes or materials.

For example, the discharge base 152 may be disposed to be partially different from the shape of the opened front surface of the main body 110 to determine the shape of the side portion 156, and may be molded with a material the same as or different from a material of the outer wall of the main body 110.

The discharge base 152 may constitute an entirety or a portion of one surface of the main body 110, for example, the front surface of the main body 110 as shown in FIG. 4, so that the center portion 154 may be defined at the center of the discharge base 152 and the side portion 156 may be defined between the outer circumferential surface of the discharge base 152 and the outer wall of the main body 110.

The discharge base 152 may be coupled to an opening of the main body 110 in various schemes, such as a scheme using a plurality of coupling ribs, and may be integrally molded with the main body 110.

In one example, as shown in FIG. 4, in an embodiment of the present disclosure, the discharge base 152 may have a shape of being indented toward an interior of the main body 110 from the side portion 156 toward the center portion 154.

A center of a front surface of the discharge base 152 may be indented toward the interior of the main body 110, so that the front surface of the discharge base 152 may form a curved surface. Accordingly, the discharge gas of the center portion 154 on the flow path of the discharge gas discharged to the gas outlet 150 may be discharged upstream from the discharge gas of the side portion 156.

When the discharge gas of the center portion 154 on the flow path of the entire discharge gas starts to be diffused prior to the discharge gas of the side portion 156, the cross-section of the discharge gas of the central portion 154 may be increased through the diffusion, and an effect in which the discharge gas of the center portion 154 with an increased cross-sectional area suppresses the discharge gas of the side portion 156 from being flowed or discharged toward the center may be increased.

Further, the front surface of the discharge base 152 constituting a portion of a space in which the discharge gas of the center portion 154 is diffused forms the curved surface, so that it may be advantageous in preventing formation of unnecessary turbulence. A curvature of the curved surface formed by the front surface of the base portion may be variously set as necessary.

In one example, an embodiment of the present disclosure may further include a guide cone disposed at a center of the center portion 154 and guiding the flow of the gas discharged through the center portion 154. The gas may be discharged between an inner surface of the center portion 154 and the guide cone.

FIG. 4 illustrates the guide cone disposed at the center of the center portion 154. As the guide cone is disposed, the discharge gas of the center portion 154 is discharged into a space between the inner surface of the center portion 154 and an outer surface of the guide cone.

When the guide cone is disposed at the center of the center portion 154, the center portion 154 may correspond to a ring-shaped discharge hole. That is, the discharge gas of the center portion 154 may have a ring-shaped cross-section and may be discharged from the center portion 154.

As described above, the discharge gas of the center portion 154 may contribute to suppressing the reduction of the cross-sectional area resulted from the discharge gas of the side portion 156 that flows toward the center in the flow process. In addition, an embodiment of the present disclosure may increase a level at which the discharge gas of the center portion 154 diffuses outward from the cross-section by disposing the guide cone at the center of the center portion 154.

When the cross-sectional area of the discharge gas of the center portion 154 is increased as the guide cone is disposed, the effect of suppressing the phenomenon in which the discharge gas of the side portion 156 flows inward of the cross-section may be increased.

In one example, in the guide cone, a rear end protruding toward the gas flow path 111 and a front end protruding in a discharge direction of the gas of the center portion 154 may respectively have conical shapes. The conical shape means a shape in which a cross-section has a circular shape and a diameter of the circle gradually decreases as a length increases.

However, in the conical shape, the circular shape may include a shape other than a definite circular shape such as an ellipse and the like, and the reduction in the diameter may not necessarily be constant, for example, a diameter reduction rate may gradually increase or gradually decrease.

Further, as the front end of the guide cone protrudes in the conical shape, an effect in which the discharge gas of the center portion 154 is concentrated toward the rim of the center portion 154 increases. Thus, the effect of suppressing the discharge gas of the side portion 156 from flowing toward the discharge gas of the center portion 154 may be further increased.

An outer circumferential surface of the guide cone may have a shape corresponding to an inner circumferential surface of the center portion 154, and a separation distance between the outer circumferential surface of the guide cone and the inner circumferential surface of the center portion 154 may be varied as needed.

Further, the guide cone may be made of a material the same as or different from the material of the discharge base 152, and a curvature of the outer surface thereof may be variously designed as needed.

In one example, the gas outlet 150 may further include a discharge guide ring. The discharge guide ring may be disposed on the inner surface of the center portion 154 and protrude in the discharge direction of the gas of the center portion 154 to guide the gas flow together with the guide cone. FIG. 4 illustrates that the guide cone and the discharge guide ring are arranged in the center portion 154.

The discharge guide ring may have a ring shape extending along the rim of the center portion 154, and may be integrally molded with the discharge base 152 or molded separately from the discharge base 152 to be coupled to the inner circumferential surface of the center portion 154.

The discharge guide ring may protrude outward from the center portion 154 or the discharge base 152 based on the gas discharge direction. The flow of the discharge gas of the center portion 154 may be concentrated between the guide cone and the discharge guide ring by the guide cone and the discharge guide ring protruding from the center portion 154.

A protruding end of the discharge guide ring may have a curved shape to facilitate the gas flow. A diameter of the discharge guide ring may be different for each portion, and the shape thereof may also be varied as needed.

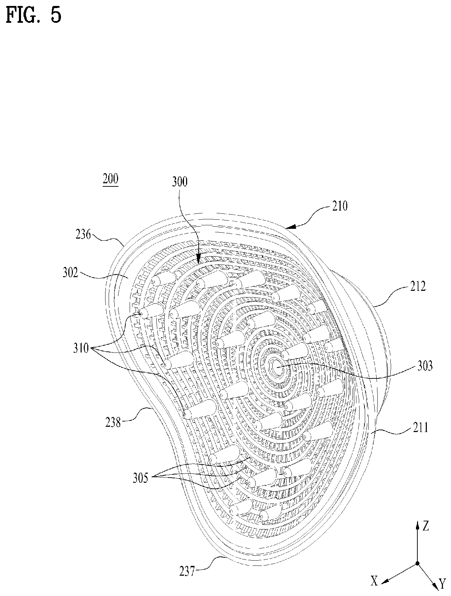

In one example, FIG. 5 shows the diffuser 200 according to an embodiment of the present disclosure. The diffuser 200 is removably coupled to the main body 110, so that the gas discharged from the gas outlet 150 is introduced into the diffuser 200 and the gas introduced into the diffuser 200 is discharged to the outside.

The hair dryer 100 according to an embodiment of the present disclosure may include the diffuser 200 as shown in FIG. 1, and the diffuser 200 may be removably coupled to the main body 110 of the hair dryer 100.

The diffuser 200 may be disposed such that gas discharged from the gas outlet 150 of the main body 110 flows into the diffuser 200. The diffuser 200 may be coupled to the main body 110 such that a rear side thereof covers the gas outlet 150, and the gas discharged from the gas outlet 150 may flow into the diffuser 200 through a gas inlet hole 215 defined at a rear side of the diffuser 200.

The user may selectively use the diffuser 200 for scalp or hair management. For example, the user may use the diffuser 200 including a massage protrusion 310 and a light irradiator 260, which will be described later, for scalp care, or may use the diffuser 200 at which a flow cross-sectional area of the gas is increased as needed in a hair drying step,
The rear side of the diffuser 200 may be coupled to the front end 112 of the main body 110.

A first coupling portion 120 may be disposed at the front end 112 of the main body 110, and a second coupling portion 220 coupled to the first coupling portion 120 may be disposed at the rear side of the diffuser 200.

A coupling scheme between the diffuser 200 and the main body 110 may vary. The diffuser 200 may be coupled to the main body 110 in a scheme such as screw coupling, fitting coupling, magnetic coupling, sliding coupling, and the like to receive the gas from the main body 110.

An embodiment of the present disclosure may improve ease of use of the user as the diffuser 200 is disposed to be removable from the main body 110. For example, the user may use the hair dryer 100 by removing the diffuser 200 when the user is desired to use the gas discharged from the gas outlet 150 of the main body 110 as it is. Further, the user may use the hair dryer 100 coupled to the diffuser 200 when the user wants a more diffused flow cross-sectional area.

The diffuser 200 includes a diffusing case 210 and a discharge cover 300, and the diffusing case 210 and a discharge cover 300 may form an exterior of the diffuser 200.

An inner diameter of the diffuser 200 may increase the inner diameter in a forward direction. That is, the diffusing case 210 of the diffuser 200 may be disposed such that an internal cross-sectional area thereof viewed from the front increases from a rear side 212 to a front side 211.

Accordingly, the gas delivered from the gas outlet 150 may be provided to the user in a state in which the flow cross-sectional area thereof is increased as the gas speed is reduced in the forward direction of the diffuser 200. The user may use the diffuser 200 for natural drying, styling, and the like of the hair.

The front side 211 of the diffusing case 210 may be opened to define an open front surface. An entirety or a portion of the front surface of the diffuser 200, that is, the diffusing case 210, may define the open surface.

The gas present inside the diffuser 200 may be discharged to the outside through the open surface of the diffusing case 210. That is, the gas inside the diffuser 200 may be provided to the user while being discharged forward through the front side 211 of the diffusing case 210.

In the diffuser 200, the open surface defined in the front side 211 of the diffusing case 210 may be exposed to the outside as it is, or the discharge cover 300 may be provided coupled to the open surface.

FIG. 5 shows a state in which the open surface is present in the front side 211 of the diffusing case 210 according to an embodiment of the present disclosure and the discharge cover 300 is coupled to the open surface.

The discharge cover 300 coupled to the open surface defined in the front side 211 of the diffusing case 210 may include a gas discharge hole 305 defined therein through which the gas may be discharged. The discharge cover 300 has a shape corresponding to the open surface of the diffusing case 210 and may be coupled to the diffusing case 210 to be located on the open surface.

A plurality of gas discharge holes 305 may be defined and may be spaced apart from each other in the front surface of the discharge cover 300. FIG. 5 shows the discharge cover 300 in which the plurality of gas discharge holes 305 are uniformly distributed and arranged in the front surface.

Accordingly, in the diffuser, the gas may be discharged through the entirety of the front surface of the discharge cover 300 and the user may receive the gas that is discharged forward of the discharge cover 300 and uniformly dispersed.

The discharge cover 300 may be disposed such that an edge 302 located on the outermost side along a radial direction of the diffuser 200 is in close contact with the diffusing case 210. That is, the diffusing case 210 may have a front circumferential portion 236 surrounding the open surface in the front side 211, and the discharge cover 300 may be disposed such that the edge 302 has a shape corresponding to the front circumferential portion 236 and in contact with the front circumferential portion 236.

As shown in FIG. 5, the diffuser 200 according to an embodiment of the present disclosure has a plurality of first portions 237 and second portions 238 on the front circumferential portion 236 of the diffusing case 210. The first portion 237 and the second portion 238 are arranged with different distances from the rear side 212 of the diffusing case 210, for example, the gas inlet hole 215 of the diffusing case 210.

In addition, the discharge cover 300 may be disposed such that the edge 302 is molded to correspond to shapes of the first portion 237 and the second portion 238 to be in close contact with the front circumferential portion 236 of the diffusing case 210.

In an embodiment of the present disclosure, the front circumferential portion 236 of the diffusing case 210 and the edge 302 of the discharge cover 300 may be designed to fit a head of the user with an arbitrary curved surface while respectively having curvatures and having different lengths protruding forward along an outer circumferential direction of the diffuser 200. Accordingly, a rate of adhesion with the scalp or the hair of the user may be efficiently increased to minimize a space between the head of the user and the diffuser 200.

Accordingly, an amount of the gas discharged forward of the discharge cover 300 or the light provided by the light irradiator 260 may be efficiently increased.

An ergonomic design is made through the front circumferential portion 236 of the diffusing case 210 and the edge 302 of the discharge cover 300 respectively arranged to form curves when viewed from the side as described above. In this case, the curvatures and the like of the front circumferential portion 236 and the edge 302 may be designed based on a standard head that is statistically determined.

For example, an embodiment of the present disclosure may define a R127 curvature design from a shape of the standard head, and design the shapes of the front circumferential portion 236 and the edge 302 to correspond thereto.

In one example, in an embodiment of the present disclosure, a proximity sensor 269 may be disposed inside the diffusing case 210 to improve ease of use and efficiency of the diffuser 200, and an open region 303 may be defined in the discharge cover 300 such that a distance measurement accuracy of the proximity sensor 269 for the target in front of the diffuser 200, for example, the hair or the scalp of the user may be improved.

That is, the proximity sensor 269 may be in various schemes such as pressure, ultrasound, infrared, and the like to measure the distance to the target in front of the proximity sensor 269, and a region of the discharge cover 300 in front of the proximity sensor 269 may be opened to define the open region 303.

In one example, FIG. 5 shows the discharge cover 300 on which a plurality of massage protrusions 310 are arranged. The massage protrusion 310 may have a pillar shape protruding forward of the diffuser 200 and may press the scalp of the user to provide a massage effect.

A cross-section shape, a protruding length, an arrangement form, and the like of the massage protrusion 310 may be variously determined in terms of a design. An embodiment of the present disclosure provides the user with scalp massage through the massage protrusion 310 while providing the gas diffused through a front surface of the discharge cover 300 to the user, thereby providing the improved ease of use and the scalp and hair care effects.

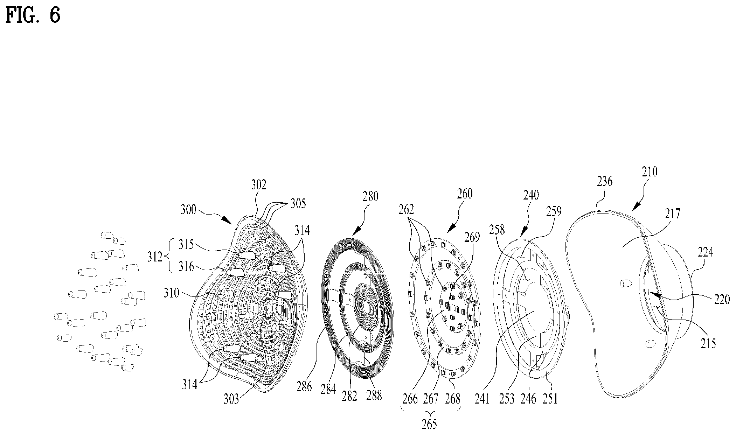

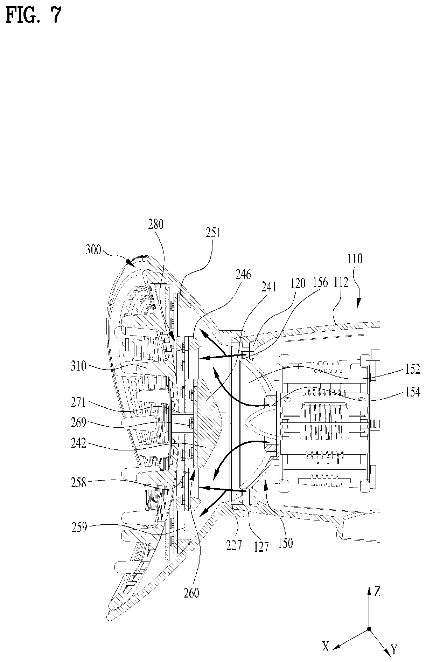

FIG. 6 is a view showing an exploded view of the diffuser 200 according to an embodiment of the present disclosure, and FIG. 7 is a view showing an internal cross-section of the diffuser 200 according to an embodiment of the present disclosure.

Referring to FIGS. 6 and 7, the diffuser 200 according to an embodiment of the present disclosure may include the diffusing case 210, a guide frame 240, the light irradiator 260, a light diffusion frame 280, and the discharge cover 300.

In the diffusing case 210, the rear side 212 may be coupled with the main body 110, and the open surface may be defined in the front side 211. The inner diameter of the diffusing case 210 may increase from the rear side 212 to the front side 211, so that the inside gas may be diffused and discharged to the outside.

That is, the gas discharged through the gas outlet 150 of the main body 110 may be provided to the user in a state in which the flow cross-sectional area thereof is increased as the gas is flowing in the diffusing case 210.

FIGS. 6 and 7 show the diffusing case 210 in which the inner diameter thereof increases from the rear side 212 to the front side 211 and accordingly an outer diameter thereof increases in the same manner.

The gas inlet hole 215 may be defined in the rear side 212 of the diffusing case 210. When the diffusing case 210 is coupled to the main body 110, the gas inlet hole 215 is positioned to face the gas outlet 150. Further, the gas discharged from the gas outlet 150 may be introduced into the diffusing case 210 through the gas inlet hole 215.

The gas inlet hole 215 may be located at a center of the rear side 212 of the diffusing case 210 when viewed from the rear, and a cross-section shape of the gas inlet hole 215 may correspond to the gas outlet 150. For example, the gas inlet hole 215 is defined to have an inner diameter larger than the side portion 156 of the gas outlet 150, so that the gas discharged from the gas outlet 150 may be completely introduced into the diffusing case 210 through the gas inlet hole 215.

The second coupling portion 220 coupled to the main body 110 may be disposed on the rear side 212 of the diffusing case 210. The diffusing case 210 may include a rear circumferential portion 217 surrounding the gas inlet hole 215 in the rear side 212, and the second coupling portion 220 may be disposed at the rear circumferential portion 217.

The second coupling portion 220 may further include a coupling sleeve 224. The coupling sleeve 224 may extend rearward from the rear circumferential portion 217. The coupling sleeve 224 may be disposed to outwardly surround the front end 112 of the main body 110.

The second coupling portion 220 may have a second magnetic fastening portion 227 embedded in the rear circumferential portion 217 or located inside the rear circumferential portion 217, and may include a power receiver disposed on an inner surface and the like of the coupling sleeve 224.

In addition, the first coupling portion 120 may be disposed at the front end 112 of the main body 110, may have a first magnetic fastening portion 127 embedded inside the outer wall of the front end 112 or located inside the outer wall, and may include a power transmitter disposed on an outer surface and the like of the outer wall of the front end 112.

The first coupling portion 120 is coupled to the second coupling portion 220. At least one of the first magnetic fastening portion 127 and the second magnetic fastening portion 227 may include a magnetic force generator, so that the first magnetic fastening portion 127 and the second magnetic fastening portion 227 may be magnetically coupled to each other. The magnetic coupling means a scheme of mutual coupling through a magnetic force generated from the magnetic force generator such as a magnet and an electromagnet.

The power transmitter may supply power to the power receiver in contact or connection with the power receiver. The power receiver may be connected to a component inside the diffuser 200, for example, the light irradiator 260 and the like to supply power thereto.

The open surface surrounded by the front circumferential portion 236 may be defined in the front side 211 of the diffusing case 210, and the gas inside the diffusing case 210 may be discharged forward of the diffuser 200 through the open surface in the front side 211.

In one example, the guide frame 240 may be disposed inside the diffusing case 210. The guide frame 240 is disposed to guide the flow of the gas introduced through the gas inlet hole 215.

The guide frame 240 may be disposed to face the gas inlet hole 215 of the diffusing case 210. The guide frame 240 may have a diffusion portion 241 at a center thereof, a first guide 246 disposed radially outward of the diffusion portion 241, and a second guide 251 disposed radially outward of the first guide 246.

The guide frame 240 may include a guide connector 253 extending along the radial direction of the diffuser 200, and the guide connector 253 may connect the diffusion portion 241, the first guide 246, and the second guide 251 to each other.

The diffusion portion 241 of the guide frame 240 is disposed to face the gas inlet hole 215 to diffuse the gas introduced through the gas inlet hole 215 outward in the radial direction. That is, the flow cross-sectional area of the gas introduced through the gas inlet hole 215 may be increased by the diffusion portion 241.

According to an embodiment of the present disclosure, in the gas outlet 150 having the center portion 154 and the side portion 156, a flow direction of the gas discharged from the center portion 154 may be changed by the diffusion portion 241. That is, the diffusion portion 241 may have a larger diameter than the center portion 154 and diffuse the gas provided from the center portion 154 outward in the radial direction.

The first guide 246 may have a ring shape, and the diffusion portion 241 may be located at a center of the first guide 246. The diffusion portion 241 may have a circular cross-section, and may be outwardly spaced apart from the diffusion portion 241 while being concentric with the diffusion portion 241 of the first guide 246.

A first flow path 258 may be located between the first guide 246 and the diffusion portion 241. That is, the first guide 246 may be spaced apart from the diffusion portion 241 to define the first flow path 258 between the first guide 246 and the diffusion portion 241. The gas diffused through the diffusion portion 241 may flow through the first flow path 258.

The second guide 251 may have a ring shape corresponding to the first guide 246, and the diffusion portion 241 and the first guide 246 may be located at a center of the second guide 251. That is, the second guide 251 may be concentric with the diffusion portion 241 and the first guide 246 and may be spaced apart from the first guide 246.

That is, an inner diameter of the first guide 246 may be larger than the diameter of the diffusion portion 241, and an inner diameter of the second guide 251 may be larger than an outer diameter of the first guide 246. Accordingly, the first flow path 258 may be defined between the diffusion portion 241 and the first guide 246, and a second flow path 259 may be defined between the first guide 246 and the second guide 251.

The gas diffused by the diffusion portion 241 may flow through the first flow path 258 and the second flow path 259. An outer diameter of the second flow path 259 may be larger than the diameter of the gas inlet hole 215, so that the gas introduced through the gas inlet hole 215 may be diffused by the diffusion portion 241 and flow with a larger flow cross-section.

The light irradiator 260 may be located in front of the guide frame 240. The light irradiator 260 may be installed on a front surface of the guide frame 240. The light irradiator 260 may have a plurality of light emitters 262 arranged on a circuit board 265. The circuit board 265 may include a plurality of circuit boards separated from each other, and the plurality of boards may be respectively arranged on the diffusion portion 241, the first guide 246, and the second guide 251 of the guide frame 240.

The light irradiator 260 may include the plurality of circuit boards 265, and the plurality of circuit boards 265 may respectively include a central board 266, a first board 267, and a second board 268.

The central board 266 may have a cross-section shape corresponding to the diffusion portion 241. For example, the diffusion portion 241 may have the circular cross-section, and the central board 266 may have a circular cross-section in the same manner as the diffusion portion 241, may be disposed on a front surface of the diffusion portion 241, and may include the plurality of light emitters 262.

The first board 267 may have a shape corresponding to the first guide 246. For example, the first guide 246 may have the ring shape, and the first board 267 may have a ring shape in the same manner as the first guide 246, may be disposed on a front surface of the first guide 246, and may include the plurality of light emitters 262.

The second board 268 may have a shape corresponding to the second guide 251. For example, the second guide 251 may have the ring shape, and the second board 268 may have a ring shape in the same manner as the second guide 251, may be disposed on a front surface of the second guide 251, and may include the plurality of light emitters 262.

The central board 266, the first board 267, and the second board 268 may be arranged to be concentric like the guide frame 240. The first board 267 may be outwardly spaced apart from the central board 266, and the second board 268 may be outwardly spaced apart from the first board 267.

That is, an inner diameter of the first board 267 may be larger than a diameter of the central board 266, and an inner diameter of the second board 268 may be larger than an outer diameter of the first board 267. Therefore, like the guide frame 240, the first flow path 258 may be located between the central board 266 and the first board 267, and the second flow path 259 may be located between the first board 267 and the second board 268.

The light irradiator 260 may irradiate light toward the front side 211 of the diffusing case 210 through the plurality of light emitters 262. The light irradiated from the light irradiator 260 may be provided to a location ahead of the diffuser 200 through the front side 211 of the diffusing case 210.

For example, the light irradiated from the light irradiator 260 may be provided to the location ahead of the diffuser 200 by passing through the open surface of the diffusing case 210, passing through the gas discharge hole 305 of the discharge cover 300, passing through the discharge cover 300, or passing through the massage protrusion 310 of the discharge cover 300.

As the light is irradiated to the location ahead of the diffuser 200, the diffuser 200 according to an embodiment of the present disclosure may perform user's hair or scalp care. The light irradiated from the light irradiator 260 may contribute to improving scalp and hair health while drying the user's scalp or hair or providing heat to the user's scalp or hair.

The proximity sensor 269 may be disposed on the circuit board 265 of the light irradiator 260. FIG. 6 shows a state in which the proximity sensor 269 is disposed on the central board 266 of the light irradiator 260 according to an embodiment of the present disclosure.

The proximity sensor 269 may be disposed at a center of the central board 266. The proximity sensor 269 may be disposed to measure the separation distance from the target positioned in front of the proximity sensor 269. The controller 115 may be disposed to control the light irradiator 260 based on the separation distance between the proximity sensor 269 and the front target measured by the proximity sensor 269.

For example, when the separation distance from the target measured by the proximity sensor 269 is equal to or less than a reference distance, the controller 115 may control the light irradiator 260 such that the light irradiator 260 irradiates the light forward. The reference distance may be predetermined in terms of a design or control.

However, the light irradiator 260 may be operated through a physical switch that is operated when the separation distance measured by the proximity sensor 269 is equal to or less than the reference distance.

**In** an embodiment of the present disclosure, as the proximity sensor 269 is used, the light irradiator 260 is operated when the separation distance from the target in front of the diffuser 200, for example, the scalp or the hair of the user is equal to or less than the reference distance, thereby improving ease of use and an operation efficiency.

The proximity sensor 269 may be disposed in various types. For example, the proximity sensor 269 may be a pressure sensor that detects whether a pressing force is applied from the user's scalp or hair, or a photosensitive sensor that measures a level at which an amount of sensed light decreases as the separation distance from the scalp or the hair decreases.

**In** addition, the proximity sensor 269 may be an **IR** sensor that measures an infrared ray transmitted from the front target, that is, the user's scalp or hair to measure the separation distance from the scalp or the hair. **In** this case, the proximity sensor 269 may be disposed to irradiate the infrared ray forward.

In one example, the light diffusion frame 280 may be located in front of the light irradiator 260. The light diffusion frame 280 may be installed on a front surface of the light irradiator 260. That is, the light diffusion frame 280 may be disposed to forwardly cover the light irradiator 260.

The light diffusion frame 280 may include a central light diffusion portion 282, a first light diffusion portion 284 and a second light diffusion portion 286. The light diffusion frame 280 may further include a light diffusion connector 288 for connecting the central light diffusion portion 282, the first light diffusion portion 284, and the second light diffusion portion 286 to each other.

The central light diffusion portion 282 may have a cross-section shape corresponding to the central board 266. For example, the central board 266 may have the circular cross-section, and the central light diffusion portion 282 may have a circular cross-section in the same manner as the central board 266 and may cover the front surface of the diffusion portion 241.

The first light diffusion portion 284 may have a shape corresponding to the first board 267. For example, the first board 267 may have the ring shape, and the first light diffusion portion 284 may have a ring shape in the same manner as the first board 267 and may cover the front surface of the first board 267.

The second light diffusion portion 286 may have a shape corresponding to the second board 268. For example, the second board 268 may have the ring shape, and the second light diffusion portion 286 may have a ring shape in the same manner as the second board 268 and may cover the front surface of the second board 268.

The central light diffusion portion 282, the first light diffusion portion 284, and the second light diffusion portion 286 may be arranged to be concentric like the guide frame 240 and the light irradiator 260. The first light diffusion portion 284 may be outwardly spaced apart from the central light diffusion portion 282, and the second light diffusion portion 286 may be outwardly spaced apart from the first light diffusion portion 284.

That is, an inner diameter of the first light diffusion portion 284 may be larger than a diameter of the central light diffusion portion 282, and an inner diameter of the second light diffusion portion 286 may be larger than an outer diameter of the first light diffusion portion 284. Accordingly, like the guide frame 240, the first flow path 258 may be located between the central light diffusion portion 282 and the first light diffusion portion 284, and the second flow path 259 may be located between the first light diffusion portion 284 and the second light diffusion portion 286.

That is, the diffuser 200 according to an embodiment of the present disclosure may be disposed in a shape in which the first flow path 258 and the second flow path 259 are extended in the front and rear direction through the guide frame 240, the light irradiator 260, and the light diffusion frame 280.

The light diffusion connector 288 may be disposed in a shape corresponding to the guide connector 253. For example, the guide connector 253 and the light diffusion connector 288 may have an extended shape along the radial direction of the diffuser 200.

The light diffusion connector 288 may be located in front of the guide connector 253. The light diffusion frame 280 may be fixed inside the diffusing case 210 as the light diffusion frame 280 is fastened to the guide connector 253.

An embodiment of the present disclosure is advantageous in terms of a design and structurally stable in that, in a state in which the guide frame 240 is constituted by a plurality of components, the plurality of components are able to be handled as a single component through the guide connector 253.

In addition, an embodiment of the present disclosure is advantageous in terms of the design and structurally stable in that, in a state in which the light diffusion frame 280 is constituted by a plurality of components, the plurality of components are able to be handled as a single component through the light diffusion connector 288.

Furthermore, the light diffusion connector 288 of the light diffusion frame 280 is coupled to the guide connector 253 of the guide frame 240, so that all of the central light diffusion portion 282, the first light diffusion portion 284, and the second light diffusion portion 286 may be stably fixed, which is advantageous in terms of coupling.

The light diffusion frame 280 may be made of a material through which light is transmitted. For example, the light diffusion frame 280 may be made of a transparent or translucent material. The light irradiated from the light irradiator 260 may be scattered and diffused while passing through the light diffusion frame 280.

In the diffuser 200 according to an embodiment of the present disclosure, the light diffusion frame 280 is disposed in front of the light irradiator 260, so that the light irradiated from the light irradiator 260 may be provided to the user while being scattered and diffused and being uniformly dispersed in a larger area.

A treatment for the diffusion or the scattering of the light may be performed on a front surface or a rear surface of the light diffusion frame 280. For example, etching may be performed or a pattern through laser processing and the like may be formed on a surface of the light diffusion frame 280.

In one example, the central light diffusion portion 282 is disposed to shield the front surface of the central board 266, and a portion of the central light diffusion portion 282 in front of the proximity sensor 269 may be opened such that the measurement of the separation distance from the target in front of the diffuser 200 by the proximity sensor 269 disposed on the central board 266 is easy.

FIG. 6 shows a state in which the proximity sensor 269 is disposed at the center of the central board 266 according to an embodiment of the present disclosure and the central light diffusion portion 282 has a hole defined at a center thereof to expose the proximity sensor 269 forwardly.

The discharge cover 300 is disposed to shield the open surface defined in the front side 211 of the diffusing case 210 in which the guide frame 240, the light irradiator 260, and the light diffusion frame 280 are embedded. The plurality of gas discharge holes 305 are defined in the discharge cover 300, so that the gas may be discharged and the light may be irradiated forward.

The discharge cover 300 may be disposed such that the edge 302 has a curvature to correspond to the front circumferential portion 236 of the diffusing case 210 when viewed from the side and is indented rearwards centerwardly when viewed from the front.

That is, a front surface of the discharge cover 300 may form a curved surface that is indented rearwards centerwardly, so that the discharge cover 300 may have a shape corresponding to the head of the user and may be optimized to provide the massage effect through the massage protrusion 310 while providing the gas and the light to the user.

The plurality of massage protrusions 310 protruding or extending forward on the front surface of the discharge cover 300 may be arranged, and a contact portion may be disposed on the surface of the discharge cover 300 such that a sense of touch with the scalp or the hair of the user may be improved and damage to the scalp and the hair may be minimized. The contact portion may be made of a material such as silicon and the like.

Some of the plurality of massage protrusions 310 may correspond to the moisture measurement protrusions 312. That is, in the diffuser 200 according to an embodiment of the present disclosure, the plurality of massage protrusions 310 may include the moisture measurement protrusions 312.

The moisture measurement protrusion 312 may be disposed to measure a moisture amount of the scalp or the hair of the user. A pair of the moisture measurement protrusions 312 may be arranged to measure an impedance, that is, bioelectrical impedance through an electric field formed therebetween.

The moisture measurement protrusion 312 may be connected to the controller 115. Further, the controller 115 may determine the impedance using a voltage, a current, a resistance, and the like, which are identified through the moisture measurement protrusion 312, determine the moisture amount of the scalp or the hair of the user based on the determined impedance, and control an operation of the fan 119, the temperature adjuster 117, or the light irradiator 260 based on the determined moisture amount.

For example, the controller 115 may control the fan 119 such that the gas speed increases as the moisture amount of the scalp or the hair of the user increases, control the temperature adjuster 117 such that the gas temperature increases, or control the light irradiator 260 such that the light amount increases.

The pair of moisture measurement protrusions 312 may be arranged. The moisture measurement protrusions 312 may include a first moisture measurement protrusion 315 electrically having a first pole and a second moisture measurement protrusion 316 having a second pole opposite to the first pole.

The controller 115 may determine the impedance and the moisture amount through the electric field formed between the first moisture measurement protrusion 315 and the second moisture measurement protrusion 316.

A plurality of pairs of moisture measurement protrusions 312, each of which is constituted by the first moisture measurement protrusion 315 and the second moisture measurement protrusion 316, may be arranged. One pair of moisture measurement protrusions 312 may be disposed to be spaced apart from another pair of moisture measurement protrusions, and different massage protrusions 310 may be positioned therebetween.

In one example, the open region 303 may be defined at the center of the discharge cover 300. The proximity sensor 269 may be exposed forward through the hole defined in the light diffusion frame 280 and the open region 303 of the discharge cover 300, and may wholly measure the separation distance from the target in front of the diffuser 200. A protection member that protects the proximity sensor 269 and allows the infrared ray or the like to pass straight therethrough may be disposed in front of the proximity sensor 269.

FIG. 7 shows an internal cross-section of the diffuser 200 and a state in which the diffuser 200 is coupled to the main body 110 to receive the gas from the gas outlet 150.

Referring to FIG. 7, in an embodiment of the present disclosure, the first coupling portion 120 of the main body 110 may include the first magnetic fastening portion 127 and the second coupling portion 220 of the diffuser 200 may include the second magnetic fastening portion 227.

The diffuser 200 may be coupled to the front end 112 of the main body 110 through the magnetic coupling between the first magnetic fastening portion 127 and the second magnetic fastening portion 227. The first coupling portion 120 may further include a hook fastener and the second coupling portion 220 may further include a hook fastened to the hook fastener, so that a coupling stability between the diffuser 200 and the main body 110 may be enhanced.

Hereinafter, the flow of the gas discharged from the gas outlet 150 according to an embodiment of the present disclosure will be described with reference to FIG. 7.

In the gas outlet 150, the gas is discharged from the center portion 154 and the side portion 156. The gas inlet hole 215 of the diffusing case 210 is defined to have a diameter equal to or larger than that of the side portion 156 and face the gas outlet 150, so that the gas discharged from the center portion 154 and the side portion 156 may be introduced into the inlet hole 215.

The guide frame 240 may be disposed inside the diffusing case 210 to face the gas outlet 150. Specifically, the diffusion portion 241 of the guide frame 240 may be positioned to face the center portion 154 of the gas outlet 150.

The gas discharged from the center portion 154 flows toward the diffusion portion 241. As the diffusion portion 241 has a diameter larger than that of the center portion 154, the gas discharged from the center portion 154 may be diffused along the radial direction of the diffuser 200.

The diffusion portion 241 may have a diffusion protrusion 242 on a rear surface thereof facing the center portion 154, and the diffusion effect of the gas discharged from the center portion 154 may be improved by the diffusion protrusion 242. The diffusion protrusion 242 may be disposed to increase in rearwardly protruding height toward a center on the cross-section when viewed from the rear.

At least a portion of the gas discharged from the center portion 154 may flow along the first flow path 258 defined between the diffusion portion 241 and the first guide 246 in the guide frame 240 by the diffusion portion 241 and the diffusion protrusion 242.

In one example, the gas discharged from the side portion 156 may have a flow form outwardly surrounding the gas discharged from the center portion 154, and the gas discharged from the side portion 156 may also diffuse outward along the radial direction of the diffuser 200 as the gas of the center portion 154 is diffused by the diffusion portion 241.

Therefore, at least a portion of the gas discharged from the side portion 156 and at least a portion of the gas discharged from the center portion 154 may flow along the second flow path 259 defined between the first guide 246 and the second guide 251 in the guide frame 240.

**In** an embodiment of the present disclosure, despite a design feature that the inner diameter of the diffuser 200 increases forwardly, the discharging of the gas discharged from the center portion 154 and the side portion 156 in the forward direction while being maintained in a specific form may be effectively suppressed through the guide frame 240.

Furthermore, in an embodiment of the present disclosure, the diffuser 200 allows the gas discharged from the center portion 154 and the side portion 156 to be effectively dispersed and diffused with a larger flow cross-sectional area while preventing the flow of the gas from being maintained in the specific form.

**In** one example, the light irradiator 260 and the light diffusion frame 280 may be arranged in front of the guide frame 240 inside the diffusing case 210. The light irradiator 260 and the light diffusion frame 280 may be coupled with the guide frame 240 and thus may be handled as a single component, so that a structure that is excellent in a space utilization and is advantageous in design may be implemented.

**In** addition, the light irradiator 260 and the light diffusion frame 280 may define the first flow path 258 and the second flow path 259 together with the guide frame 240. **In an** embodiment of the present disclosure, as the structure in which the light irradiator 260 and the light diffusion frame 280 define the first flow path 258 and the second flow path 259 together with the guide frame 240 is achieved, the flow of the gas formed by the guide frame 240 may be effectively maintained and the gas may be discharged forwardly of the diffuser 200 through the light irradiator 260 and the light diffusion frame 280.

**In** one example, in the light irradiator 260, the first board 267 may be positioned forwardly of the central board 266, and the second board 268 may be positioned forwardly of the first board 267. Accordingly, the plurality of light emitters 262 arranged in the light irradiator 260 may be arranged to form a spherical surface that is substantially indented rearward.

Accordingly, the plurality of light emitters 262 may be arranged in a form in which a distance from a center of the light irradiator 260 along the radial direction increases forwardly. Such arrangement of the light emitters 262 may correspond to the shape of the front surface of the discharge cover 300 indented rearward.

That is, in an embodiment of the present disclosure, the plurality of light emitters 262 arranged on the light irradiator 260 are arranged to form the curved surface to correspond to the user's head having the curvature, so that a uniform amount of light may be provided to the user's scalp and hair.

Like the light irradiator 260, the guide frame 240 may be disposed such that the first guide 246 is positioned forwardly of the diffusion portion 241 and the second guide 251 is positioned forwardly of the first guide 246.

Accordingly, the first board 267 disposed on the front surface of the first guide 246 may be positioned forwardly of the central board 266 disposed on the front surface of the diffusion portion 241, and the second board 268 disposed on the front surface of the second guide 251 may be positioned forwardly of the first board 267.

Like the light irradiator 260, in the light diffusion frame 280, the first light diffusion portion 284 may be positioned forwardly of the central light diffusion portion 282 and the second light diffusion portion 286 may be positioned forwardly of the first light diffusion portion 284. Accordingly, a distance between the light diffusion frame 280 and the light irradiator 260 may be kept constant, and uniform dispersion and scattering of the light may be induced.

**In** addition, in the guide frame 240, as the second guide 251 is positioned forwardly of the first guide 246 and the first guide 246 is positioned forwardly of the diffusion portion 241, a space in which the gas introduced from the gas inlet hole 215 is diffused in the radial direction may be secured and the gas may be smoothly introduced into the first flow path 258 and the second flow path 259.

FIG. 7 shows the guide frame 240, the light irradiator 260, and the light diffusion frame 280 protruding forward in a direction away from centers thereof, according to an embodiment of the present disclosure.

**In** one example, FIG. 7 shows a light blocking portion 271 surrounding the proximity sensor 269. The light blocking portion 271 may be disposed to surround the proximity sensor 269 along the circumferential direction of the diffuser 200, thereby preventing a situation in which the light emitter 262 around the proximity sensor 269 affects the proximity sensor 269.

**In** addition, the light blocking portion 271 may be opened forward to prevent structural interference from occurring in a measurement of the separation distance between the diffuser 200 and the front target by the proximity sensor 269. For example, when the proximity sensor 269 measures the infrared ray transmitted from the target, the light blocking portion 271 is opened forward to allow the infrared ray transmitted from the target to be completely provided to the proximity sensor 269.

The light blocking portion 271 may be formed in a hollow cylindrical shape. The proximity sensor 269 may be located inside the light blocking portion 271. The light blocking portion 271 may have a shape extending from the central board 266 to the discharge cover 300.

The light blocking portion 271 may be disposed to extend rearward from the discharge cover 300, or may be formed integrally with the discharge cover 300 or integrally with the central board 266. The light blocking portion 271 may be manufactured separately from the discharge cover 300 and the central board 266, and may be coupled or connected to the discharge cover 300 and/or the central board 266.

In one example, as described above, the hair dryer 100 according to an embodiment of the present disclosure may include the main body 110, the handle 180, and the diffuser 200.

The main body 110 may include the gas outlet 150 for discharging the gas introduced from the outside, and the handle 180 may extend from the main body 110. The diffuser 200 may be removably coupled to the main body 110, so that the gas discharged from the gas outlet 150 may flow into the diffuser 200 and the gas introduced into the diffuser 200 may be discharged to the outside.

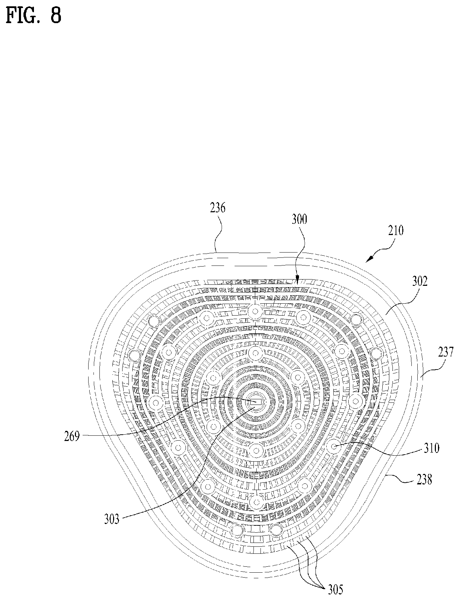

In one example, FIG. 8 shows a view of the diffuser 200 according to an embodiment of the present disclosure viewed from the front.

The diffuser 200 may include the diffusing case 210. In the diffusing case 210, the rear side 212 may be coupled to the main body 110, the gas discharged from the gas outlet 150 may be introduced into the diffusing case 210 through the gas inlet hole 215 defined in the rear side 212. The open surface may be defined in the front side 211, so that the gas introduced into the diffusing case 210 may be discharged to the outside through the open surface.

The diffusing case 210 may include the front circumferential portion 236 surrounding the open surface, and the front circumferential portion 236 may include the first portion 237 and the second portion 238, and at least a portion of the first portion 237 may be positioned forwardly of the second portion 238.

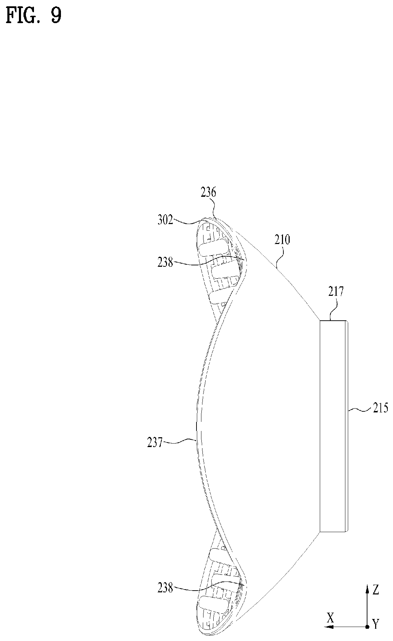

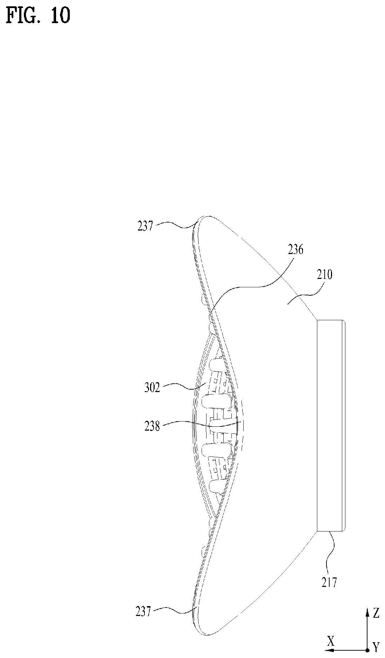

FIGS. 9 and 10 are views of the diffuser 200 according to an embodiment of the present disclosure viewed from the side. Specifically, FIG. 9 shows the first portion 237 of the diffuser 200 according to an embodiment of the present disclosure, and FIG. 10 shows the second portion 238 of the diffuser 200 according to an embodiment of the present disclosure.

Referring to FIGS. 8 to 10, in an embodiment of the present disclosure, the diffusing case 210 may include the front circumferential portion 236 surrounding the front surface, that is, the open surface, and the front circumferential portion 236 may include the first portion 237 and the second portion 238.

The first portion 237 and the second portion 238 may be arranged forward of the gas inlet hole 215 with different distances to the gas inlet hole 215. For example, the first portion 237 may be positioned forwardly of the second portion 238.

FIG. 8 shows the first portion 237 positioned forwardly of the second portion 238 and the second portion 238 positioned rearwardly of the first portion 237. In the present disclosure, the first portion 237 and the second portion 238 of the front circumferential portion 236 may be distinguished and defined in a relative relationship therebetween.

The diffuser 200 may discharge the gas to the user while being positioned adjacent to the user's scalp and hair. However, the user's head is not flat. **In** a general body, the head may be understood to be in a shape of a sphere having a curvature.

The diffusing case 210 may provide the gas to the user through the front surface thereof corresponding to the open surface. When the open surface is flat, that is, when the open surface has a straight shape at a side when viewed from the side as shown in FIGS. 9 and 10, it may be difficult for an entirety of the diffusing case 210 to be in close contact with the user's head corresponding to the sphere surface.

For example, when a central side of the open surface is in close contact with or adjacent to the user's head, an edge of the open surface may be spaced apart from the user's scalp and hair. That is, a distance between the central side of the open surface and the user's scalp may be greater than a distance between the edge of the open surface and the user's scalp.

Accordingly, the gas discharged through the open surface may be discharged to the outside through a space defined between the edge of the open surface and the scalp and the hair of the user and may be lost, and the care effect and the like using the diffuser 200 may be reduced.

Consequently, when the open surface from which the gas is discharged is flat, a deviation in a separation distance from the scalp and the hair of the user may occur at positions of the open surface. Accordingly, an embodiment of the present disclosure may design the open surface to correspond to the curved surface corresponding to the user's head.

**In** a case in which the open surface is designed to form the curved surface as described above, when the front circumferential portion 236 corresponding to the edge of the open surface in the diffusing case 210 is located on one plane, it may be disadvantageous for the open surface to form the curved surface.

That is, in an embodiment of the present disclosure, in order for the open surface defined in the front side 211 of the diffusing case 210 to form the curved surface, an entirety of the front circumferential portion 236 may not be positioned on one plane and the front circumferential portion 236 may be divided into the first portion 237 and the second portion 238 having protruding heights in the forward direction different from each other.

The position comparison between the first portion 237 and the second portion 238 as described above may be identified by comparing positions of a line formed by a front end of the first portion 237 and a line formed by a front end of the second portion 238 when viewed from the side.

For example, when the diffuser 200 is viewed from the side as shown in FIGS. 9 and 10, the line formed by the front end of the first portion 237 may be positioned forwardly of the line formed by the front end of the second end 238, and the line formed by the front end of the second portion 238 may be positioned rearwardly of the line formed by the front end of the first portion 237.

That is, the present disclosure may form the front circumferential portion 236 with the first portion 237 and the second portion 238 at different positions in the front and rear direction for the open surface to easily form the curved surface, for example, a sphere surface.

As shown in FIG. 8, the second portion 238 positioned rearwardly of the first portion 237 in the front and rear direction is positioned closer to a center of a circle than a general circumference of the circle, and the first portion 237 is located farther from the center of the circle when compared to the second portion 238.

As described above, an embodiment of the present disclosure may allow the open surface to effectively form the curved surface and allow an entirety of the front circumferential portion 236 to be at a uniform distance from the scalp of the user as the front circumferential portion 236 of the diffusing case 210 is not in a general circular shape when viewed from the front and is not in a general straight shape when viewed from the side.

In one example, as shown in FIG. 8, in an embodiment of the present disclosure, the front circumferential portion 236 is constituted by a plurality of first portions 237 and a plurality of second portions 238, and the first portion 237 and the second portion 238 are alternately arranged along a circumference of the open surface.

As shown in FIGS. 9 and 10, when viewed from the side, the first portion 237 may be a convex portion and the second portion 238 may be a concave portion.

In an embodiment of the present disclosure, the first portion 237 and the second portion 238 may be alternately arranged without a directionality in order to provide the same effect in any use direction of the user. **In** addition, the front circumferential portion 236 may correspond to the general sphere surface through the alternating shape as described above, so that the entirety of the front circumferential portion 236 may be in close contact with the sphere surface.

In one example, in an embodiment of the present disclosure, the first portion 237 and the second portion 238 may extend along the circumference of the open surface while at least partially varying in a length forwardly extending from the gas inlet hole 215.

That is, the first portion 237 and the second portion 238 may be arranged such that front ends of at least portions thereof form a curved surface when viewed from the side. The first portion 237 and the second portion 238 may have shapes capable of being in close contact with the sphere surface while lengths thereof extending forward from the gas inlet hole 215, the rear side of the diffuser 200, the main body 110, and the like are continuously changing.

In one example, in an embodiment of the present disclosure, the first portion 237 and the second portion 238 may extend along the circumference of the open surface while respectively forming curvatures when viewed from the side.

As described above, the head of the body may have the surface corresponding to the sphere surface, and the first portion 237 and the second portion 238 may extend along the circumference of the open surface while respectively forming the curvatures to correspond to the sphere surface as described above.

That is, the first portion 237 and the second portion 238 extend along the circumference of the open surface while the lines formed by the front ends of the first portion 237 and the second portion 238 respectively form curves with curvatures when viewed from the side. The curvatures formed by the first portion 237 and the second portion 238 may not always be constant and may be variously determined in terms of design to correspond to the sphere surface.

FIGS. 9 and 10 show the first portion 237 and the second portion 238 respectively having the curvatures and extending along the circumference of the open surface, according to an embodiment of the present disclosure.

In one example, in an embodiment of the present disclosure, the second portion 238 may have a concavely curved shape and may be disposed such that a forwardly extended length thereof increases in a direction away from a center thereof.

FIG. 10 shows the second portion 238 according to an embodiment of the present disclosure. Referring to FIG. 10, the second portion 238 may have the concavely curved shape in the front and rear direction.

However, the concavely curved shape may correspond to the shape of the front end of the second portion 328 viewed from the side. When viewed from the front, the concavely curved second portion 238 may not be indented into the diffusing case 210.

That is, it may be understood that a cross-section of the diffusing case 210 may be maintained in the circular shape when viewed from the front, the second portion 238 is not indented toward a center of the diffusing case 210 when viewed from the front, but is disposed such that only the forwardly extended length, that is, the shape formed by the front end of the second portion 238 forms the curve, and the line formed by the front end of the second portion 238 is concavely curved to form the curve.

This is because the shape characteristics of the first portion 237 or the second portion 238 are derived three-dimensionally. It may be understood in FIG. 8 that the second portion 238 is indented toward a center of the cross-section of the diffused case 210 than the first portion 237, but this is particularly because it is a shape viewed from the front. In fact, in the second portion 238, the front end thereof merely forms the curve through the change in the extended length of the front end, and the second portion 238 may not be indented toward the center of the diffusion case 210 than the first portion 237 on a specific plane viewed from the front.

In relation to the second portion 238, when the user's head is regarded to have the sphere surface, due to three-dimensional characteristics, a user's head portion corresponding to the second portion 238 may protrude more than a portion corresponding to the first portion 237 even when the first portion 237 is in close contact with the user's head.

Accordingly, in an embodiment of the present disclosure, the second portion 238 is disposed to be more concavely curved than the first portion 237. Accordingly, not only the first portion 237, but also the second portion 238 may be in close contact with the user's head, which is regarded as the sphere surface. This may be effective in providing the gas, irradiating the light, or performing the massage.

In one example, in an embodiment of the present disclosure, the first portion 237 may have the convexly curved shape and may be disposed such that a forwardly extended length thereof increases in a direction toward a center thereof.

FIG. 9 shows the first portion 237 having the convexly curved shape in a forward direction according to an embodiment of the present disclosure. Each first portion 237 may be located between a pair of second portions 238, and each second portion 238 may also be located between a pair of first portions 237.

Like the second portion 238, in the first portion 237, the convexly curved shape may correspond to the shape of the front end of the first portion 237 viewed from the side. When viewed from the front, the convexly curved the first portion 237 may not protrude outwardly of the diffusing case 210. Such characteristics of the first portion 237 and the second portion 238 may be identified through the shape of the diffusing case 210 in FIGS. 9 and 10.

In relation to the first portion 237, when the user's head is regarded to have the sphere surface, due to three-dimensional characteristics, the user's head portion corresponding to the first portion 237 may be indented more than the portion corresponding to the second portion 238 even when the second portion 238 is in close contact with the user's head.

Accordingly, in an embodiment of the present disclosure, the first portion 237 is disposed to be more convexly curved than the second portion 238. Accordingly, the first portion 237 is able to be three-dimensionally attached to the user's head, which is regarded as the sphere surface. This may be effective in providing the gas, irradiating the light, or performing the massage.

The first portion 237 may be disposed such that the forwardly extended length thereof increases in a direction toward a center of the first portion 237 in a circumferential direction of the front circumferential portion 236 when viewed from the side, so that the first portion 237 may have the convex shape.

The second portion 238 may be disposed such that the forwardly extended length thereof decreases in a direction toward a center of the second portion 238 in the circumferential direction of the front circumferential portion 236 when viewed from the side, so that the second portion 238 may have the concave shape.

The first portion 237 and the second portion 238 may be arranged such that the curvatures thereof are constant and the forwardly extended lengths thereof vary along the circumference of the front circumferential portion 236, or may be arranged such that the curvatures thereof vary along the circumference.

FIGS. 9 and 10 show the first portion 237 and the second portion 238 of substantially triangular shapes in which vertex portions are rounded when viewed from the side, according to an embodiment of the present disclosure.

Specifically, FIG. 9 shows that the first portion 237 with a central side located at the frontmost position has the substantially triangular shape. FIG. 10 shows that the concave shape of the second portion 238 with a central side located at the rearmost position has the substantially triangular shape.

As described above, the first portion 237 and the second portion 238 in an embodiment of the present disclosure may have various shapes optimized for close contact with the user's head.

**In** one example, an embodiment of the present disclosure may further include the light irradiator 260 disposed inside the diffusing case 210 and irradiating the light to the front side 211 of the diffusing case 210 through the open surface.

When the open surface of the diffusing case 210 is defined in a flat shape, and when the light irradiator 260 irradiates the light toward the user's head, a portion of the front circumferential portion 236 may be in a close contact with or adjacent to the user's head, **but a** remaining portion thereof may be spaced apart from the user's head.

Through such separation space, a portion of the light irradiated from the light irradiator 260 may leak out and be lost, thereby reducing the user's scalp and hair care effects.

However, as in an embodiment of the present disclosure, an entirety of the front circumferential portion 236 where the forwardly convex first portion 237 and the rearwardly concave second portion 238 are formed may be uniformly in close contact with or adjacent to the user's head. Therefore, the care effect may be improved by minimizing the situation in which the light irradiated from the light irradiator 260 leaks to the outside and is lost.

In one example, in an embodiment of the present disclosure, the diffuser 200 includes the discharge cover 300. The discharge cover 300 is coupled to the front side 211 of the diffusing case 210 to shield the open surface, and may include the gas discharge hole 305 through which the gas inside the diffusing case 210 is discharged to the outside.

That is, in an embodiment of the present disclosure, the gas transmitted from the main body 110 or the light irradiated from the light irradiator 260 may be supplied to the user's scalp and hair through the discharge cover 300.

In one example, the discharge cover 300 may include the massage protrusion 310 that protrudes forward to press the target in front of the discharge cover 300.

That is, in an embodiment of the present disclosure, the diffuser 200 may massage the user's scalp through the massage protrusion 310. As the front circumferential portion 236 of the diffusing case 210 has the first portion 237 and the second portion 238, a level of close contact between the discharge cover 300 and the user's head may be improved, and the massage effect may also be increased.

In one example, in an embodiment of the present disclosure, the discharge cover 300 may include the edge 302 having a curvature corresponding to the front circumferential portion 236 when viewed from the side and located inward of the front circumferential portion 236.

That is, the entirety of the discharge cover 300 may be in close contact with the user's head with the front circumferential portion 236 as the edge 302 located on an inner surface of the diffusing case 210 has a shape corresponding to the front circumferential portion 236 including the first portion 237 and the second portion 238.

In one example, the discharge cover 300 has a shape in which a front surface thereof directed in the front direction is indented rearwards centerwardly. When the discharge cover 300 is disposed in the diffuser 200, the discharge cover 300 may become means for defining the shape of the aforementioned open surface.

That is, the discharge cover 300 may have a spherical surface shape that is intended rearwards centerwardly. Further, as the edge 302 of the discharge cover 300 and the front circumferential portion 236 of the diffusing case are arranged to be entirely in close contact with the sphere surface through the formation of the first portion 237 and the second portion 238, the edge 302 as well as the front surface of the discharge cover 300 may be in close contact with the user's head corresponding to the sphere surface, so that the effects of the massage, the gas discharge, and the light irradiation may be improved.

Although a specific embodiment of the present disclosure has been illustrated and described above, those of ordinary skill in the art to which the present disclosure pertains will appreciate that various modifications are possible within the limits without departing from the technical scope of the present disclosure provided by the following claims.

## Claims

1. A diffuser comprising:
a diffusing case (210) having a rear side (212) removably coupled to a main body (110) of a hair dryer (100) and having a truncated cone shape such that a diameter increases from a rear to a front, wherein gas discharged from the main body (110) is introduced into the diffusing case (210) through a gas inlet hole (215) defined at the rear side (212), wherein an open surface is located at a front side (211) of the diffusing case (210) such that the gas introduced into the diffusing case (210) is discharged to outside through the open surface, and
a discharge cover (300) coupled to the front side (211) of the diffusing case (210) to shield the open surface and has a front surface directed in a forward direction having a shape of being indented rearwards in a direction toward a center of the front surface,
wherein the diffusing case (210) includes a front circumferential portion (236) surrounding the open surface,
**characterized in that** the front circumferential portion (236) includes a plurality of first portions (237) and a plurality of second portions (238) located at least partially backwardly from the first portions (237),
wherein a length extending forward of at least a portion of the first portion (237) and the second portion (238) is changed along the circumference of the open surface, and
wherein the plurality of first portions (237) and the plurality of second portions (238) are alternately arranged with each other along a circumference of the open surface and extend along the circumference of the open surface while respectively having curvatures when viewed in a side direction.

2. The diffuser of claim 1, wherein each second portion (238) is curved concavely in a rearward direction, wherein the distance from the front end of each second portion (238) to the rear side increases in a direction away from a center of the second portion (238).

3. The diffuser of claim 2, wherein the first portion (237) has a convex curved shape in a forward direction, wherein the distance from the front end of each first portion (237) to the rear side increases in a direction toward a center of the first portion (237).

4. The diffuser of claim 1, further comprising:
a light irradiator (260) disposed inside the diffusing case (210) and irradiating light forwardly of the diffusing case (210) through the open surface.

5. The diffuser of claim 1, wherein the discharge cover (300) includes a gas discharge hole (305) defined therein for discharging the gas inside the diffusing case (210) to the outside.

6. The diffuser of claim 5, wherein the discharge cover (300) includes a massage protrusion (310) protruding forward to press a target in front of the discharge cover (300).

7. The diffuser of claim 5, wherein the discharge cover (300) includes an edge having a curvature corresponding to the front circumferential portion when viewed from the side, wherein the edge is located inward of the front circumferential portion.

8. A hair dryer comprising:
a main body (110) including a gas outlet (150) for discharging gas therethrough;
a handle (180) extending from the main body (110); and
a diffuser (200) removably coupled to the main body (110) to introduce the gas discharged from the gas outlet (150) therein and discharge the gas introduced therein to outside,
wherein the diffuser (200) includes:
a diffusing case (210) having a rear side (212) removably coupled to the main body (110) of the hair dryer and having a truncated cone shape such that a diameter increases from a rear to a front, wherein gas discharged from the gas outlet (150) is introduced into the diffusing case (210) through a gas inlet hole (215) defined at the rear side (212), wherein an open surface is defined at a front side (211) of the diffusing case (210) such that the gas introduced into the diffusing case (210) is discharged to outside through the open surface, and
a discharge cover (300) coupled to the front side (211) of the diffusing case (210) to shield the open surface and has a front surface directed in a forward direction having a shape of being indented rearwards in a direction toward a center of the front surface,
wherein the diffusing case (210) includes a front circumferential portion (236) surrounding the open surface,
**characterized in that** the front circumferential portion (236) includes a plurality of first portions (237) and a plurality of second portions (238) located at least partially backwardly from the first portions (237),
wherein a length extending forward of at least a portion of the first portion (237) and the second portion (238) is changed along the circumference of the open surface, and
wherein the plurality of first portions (237) and the plurality of second portions (238) are alternately arranged with each other along a circumference of the open surface and extend along the circumference of the open surface while respectively having curvatures when viewed in a side direction.

9. The hair dryer of claim 8, wherein a distance from a front end of each of the first portion (237) and the second portion (238) to the rear side (212) varies along at least a partial region of the front end.

10. The hair dryer of claim 8, wherein each second portion (238) has a concave curved shape in a rearward direction, wherein the distance from the front end of each second portion (238) to the rear side (212) increases in a direction away from a center of the second portion (238),
wherein each first portion (237) has a convex curved shape in a forward direction, wherein the distance from the front end of each first portion (237) to the rear side (212) increases in a direction toward a center of the first portion (237).

## Patentansprüche

1. Diffusor, aufweisend:
ein Diffusorgehäuse (210), das eine Rückseite (212) aufweist, die lösbar an einen Hauptkörper (110) eines Haartrockners (100) gekoppelt ist, und eine Kegelstumpfform aufweist, so dass ein Durchmesser von hinten nach vorn zunimmt, wobei Gas, das aus dem Hauptkörper (110) ausgeleitet wird, durch eine an der Rückseite (212) ausgebildete Gaseinlassöffnung (215) in das Diffusorgehäuse (210) eingeführt wird, wobei sich an einer Vorderseite (211) des Diffusorgehäuses (210) eine Offenfläche befindet, so dass das in das Diffusorgehäuse (210) eingeführte Gas durch die Offenfläche nach außen ausgeleitet wird, und
eine Austrittsabdeckung (300), die an die Vorderseite (211) des Diffusorgehäuses (210) gekoppelt ist, um die Offenfläche abzuschirmen, und eine in Vorwärtsrichtung gerichtete Vorderfläche aufweist, die die Form hat, nach hinten in einer Richtung zu einem Zentrum der Vorderfläche eingedrückt zu sein,
wobei das Diffusorgehäuse (210) einen die Offenfläche umgebenden vorderen Umfangsabschnitt (236) aufweist,
**dadurch gekennzeichnet, dass** der vordere Umfangsabschnitt (236) mehrere erste Abschnitte (237) und mehrere zweite Abschnitte (238) aufweist, die zumindest teilweise rückwärtig von den ersten Abschnitten (237) angeordnet sind,
wobei eine nach vorn sich erstreckende Länge zumindest eines Teils des ersten Abschnitts (237) und des zweiten Abschnitts (238) entlang des Umfangs der Offenfläche verändert ist, und
wobei die mehreren ersten Abschnitte (237) und die mehreren zweiten Abschnitte (238) abwechselnd zueinander entlang eines Umfangs der Offenfläche angeordnet sind und sich entlang des Umfangs der Offenfläche erstrecken, während sie jeweils Krümmungen aufweisen, wenn sie in einer Seitenrichtung betrachtet werden.

2. Diffusor nach Anspruch 1, wobei jeder zweite Abschnitt (238) konkav in einer Rückwärtsrichtung gekrümmt ist, wobei der Abstand vom vorderen Ende jedes zweiten Abschnitts (238) zur Rückseite in einer Richtung weg von einem Zentrum des zweiten Abschnitts (238) zunimmt.

3. Diffusor nach Anspruch 2, wobei der erste Abschnitt (237) eine konvex gekrümmte Form in einer Vorwärtsrichtung aufweist, wobei der Abstand vom vorderen Ende jedes ersten Abschnitts (237) zur Rückseite in einer Richtung zu einem Zentrum des ersten Abschnitts (237) zunimmt.

4. Diffusor nach Anspruch 1, ferner aufweisend:
einen Lichtstrahler (260), der innerhalb des Diffusorgehäuses (210) angeordnet ist und Licht vorwärts vom Diffusorgehäuse (210) durch die Offenfläche abstrahlt.

5. Diffusor nach Anspruch 1, wobei die Austrittsabdeckung (300) eine darin ausgebildete Gasaustrittsöffnung (305) aufweist, zum Ausleiten des Gases innerhalb des Diffusorgehäuses (210) nach außen.

6. Diffusor nach Anspruch 5, wobei die Austrittsabdeckung (300) einen Massagevorsprung (310) aufweist, der nach vorn vorspringt, um ein Ziel vor der Austrittsabdeckung (300) zu drücken.

7. Diffusor nach Anspruch 5, wobei die Austrittsabdeckung (300) eine Kante aufweist, die eine Krümmung entsprechend dem vorderen Umfangsabschnitt aufweist, wenn sie von der Seite betrachtet wird, wobei die Kante innerhalb des vorderen Umfangsabschnitts angeordnet ist.

8. Haartrockner, aufweisend:
einen Hauptkörper (110), der einen Gasauslass (150) zum Ausleiten von Gas aufweist;
einen Griff (180), der sich von dem Hauptkörper (110) erstreckt; und
einen Diffusor (200), der lösbar an den Hauptkörper (110) gekoppelt ist, um das aus dem Gasauslass (150) ausgeleitete Gas darin einzuführen und das darin eingeführte Gas nach außen auszuleiten,
wobei der Diffusor (200) aufweist:
ein Diffusorgehäuse (210), das eine Rückseite (212) aufweist, die lösbar an den Hauptkörper (110) des Haartrockners gekoppelt ist, und eine Kegelstumpfform aufweist, so dass ein Durchmesser von hinten nach vorn zunimmt, wobei Gas, das aus dem Gasauslass (150) ausgeleitet wird, durch eine an der Rückseite (212) ausgebildete Gaseinlassöffnung (215) in das Diffusorgehäuse (210) eingeführt wird, wobei an einer Vorderseite (211) des Diffusorgehäuses (210) eine Offenfläche ausgebildet ist, so dass das in das Diffusorgehäuse (210) eingeführte Gas durch die Offenfläche nach außen ausgeleitet wird, und
eine Austrittsabdeckung (300), die an die Vorderseite (211) des Diffusorgehäuses (210) gekoppelt ist, um die Offenfläche abzuschirmen, und eine in Vorwärtsrichtung gerichtete Vorderfläche aufweist, die die Form hat, nach hinten in einer Richtung zu einem Zentrum der Vorderfläche eingedrückt zu sein,
wobei das Diffusorgehäuse (210) einen die Offenfläche umgebenden vorderen Umfangsabschnitt (236) aufweist,
**dadurch gekennzeichnet, dass** der vordere Umfangsabschnitt (236) mehrere erste Abschnitte (237) und mehrere zweite Abschnitte (238) aufweist, die zumindest teilweise rückwärtig von den ersten Abschnitten (237) angeordnet sind,
wobei eine nach vorn sich erstreckende Länge zumindest eines Teils des ersten Abschnitts (237) und des zweiten Abschnitts (238) entlang des Umfangs der Offenfläche verändert ist, und
wobei die mehreren ersten Abschnitte (237) und die mehreren zweiten Abschnitte (238) abwechselnd zueinander entlang eines Umfangs der Offenfläche angeordnet sind und sich entlang des Umfangs der Offenfläche erstrecken, während sie jeweils Krümmungen aufweisen, wenn sie in einer Seitenrichtung betrachtet werden.

9. Haartrockner nach Anspruch 8, wobei ein Abstand von einem vorderen Ende jedes der ersten Abschnitte (237) und der zweiten Abschnitte (238) zur Rückseite (212) entlang zumindest eines Teilbereichs des vorderen Endes variiert.

10. Haartrockner nach Anspruch 8, wobei jeder zweite Abschnitt (238) eine konkav gekrümmte Form in einer Rückwärtsrichtung aufweist, wobei der Abstand vom vorderen Ende jedes zweiten Abschnitts (238) zur Rückseite (212) in einer Richtung weg von einem Zentrum des zweiten Abschnitts (238) zunimmt,
wobei jeder erste Abschnitt (237) eine konvex gekrümmte Form in einer Vorwärtsrichtung aufweist,
wobei der Abstand vom vorderen Ende jedes ersten Abschnitts (237) zur Rückseite (212) in einer Richtung zu einem Zentrum des ersten Abschnitts (237) zunimmt.

## Revendications

1. Diffuseur, comprenant :
un boîtier de diffuseur (210) ayant une face arrière (212) accouplée de manière détachable à un corps principal (110) d'un sèche-cheveux (100), et ayant une forme de tronc de cône de sorte qu'un diamètre augmente de l'arrière vers l'avant, un gaz déchargé depuis le corps principal (110) étant introduit dans le boîtier de diffuseur (210) par un orifice d'entrée de gaz (215) formé sur la face arrière (212), une surface ouverte étant formée sur une face avant (211) du boîtier de diffuseur (210) de sorte que le gaz introduit dans le boîtier de diffuseur (210) soit déchargé vers l'extérieur à travers la surface ouverte ; et
un couvercle de sortie (300) accouplé à la face avant (211) du boîtier de diffuseur (210) afin d'écranter la surface ouverte, et ayant une surface avant orientée vers l'avant ayant une forme enfoncée vers l'arrière dans une direction vers un centre de la surface avant,
le boîtier de diffuseur (210) comprenant une portion périphérique avant (236) entourant la surface ouverte,
**caractérisé en ce que** la portion périphérique avant (236) comprend une pluralité de premières portions (237) et une pluralité de secondes portions (238) disposées au moins partiellement en arrière des premières portions (237),
une longueur s'étendant vers l'avant d'au moins une partie de la première portion (237) et de la seconde portion (238) étant modifiée le long de la périphérie de la surface ouverte, et
la pluralité des premières portions (237) et la pluralité des secondes portions (238) étant disposées alternativement l'une par rapport à l'autre le long d'une périphérie de la surface ouverte et s'étendant le long de la périphérie de la surface ouverte, tout en ayant chacune des courbures lorsqu'elles sont vues dans une direction latérale.

2. Diffuseur selon la revendication 1, dans lequel chaque seconde portion (238) est courbée de manière concave dans une direction arrière, une distance depuis une extrémité avant de chaque seconde portion (238) jusqu'à la face arrière augmentant dans une direction s'éloignant d'un centre de la seconde portion (238).

3. Diffuseur selon la revendication 2, dans lequel la première portion (237) a une forme courbée de manière convexe dans une direction avant, une distance depuis une extrémité avant de chaque première portion (237) jusqu'à la face arrière augmentant dans une direction vers un centre de la première portion (237).

4. Diffuseur selon la revendication 1, comprenant en outre :
un émetteur de lumière (260) disposé à l'intérieur du boîtier de diffuseur (210) et émettant une lumière vers l'avant depuis le boîtier de diffuseur (210) à travers la surface ouverte.

5. Diffuseur selon la revendication 1, dans lequel le couvercle de sortie (300) comprend un orifice de sortie de gaz (305) formé en celui-ci, destiné à décharger vers l'extérieur le gaz à l'intérieur du boîtier de diffuseur (210).

6. Diffuseur selon la revendication 5, dans lequel le couvercle de sortie (300) comprend une saillie de massage (310) faisant saillie vers l'avant afin de presser une cible située devant le couvercle de sortie (300).

7. Diffuseur selon la revendication 5, dans lequel le couvercle de sortie (300) comprend un bord ayant une courbure correspondant à la portion périphérique avant lorsqu'il est vu de côté, le bord étant disposé à l'intérieur de la portion périphérique avant.

8. Sèche-cheveux, comprenant :
un corps principal (110) comprenant une sortie de gaz (150) destinée à y décharger un gaz ;
une poignée (180) s'étendant depuis le corps principal (110) ; et
un diffuseur (200) accouplé de manière détachable au corps principal (110) afin d'y introduire le gaz déchargé depuis la sortie de gaz (150) et de décharger vers l'extérieur le gaz ainsi introduit,
le diffuseur (200) comprenant :
un boîtier de diffuseur (210) ayant une face arrière (212) accouplée de manière détachable au corps principal (110) du sèche-cheveux, et ayant une forme de tronc de cône de sorte qu'un diamètre augmente de l'arrière vers l'avant, le gaz déchargé depuis la sortie de gaz (150) étant introduit dans le boîtier de diffuseur (210) par un orifice d'entrée de gaz (215) formé sur la face arrière (212), une surface ouverte étant formée sur une face avant (211) du boîtier de diffuseur (210) de sorte que le gaz introduit dans le boîtier de diffuseur (210) soit déchargé vers l'extérieur à travers la surface ouverte ; et
un couvercle de sortie (300) accouplé à la face avant (211) du boîtier de diffuseur (210) afin d'écranter la surface ouverte, et ayant une surface avant orientée vers l'avant ayant une forme enfoncée vers l'arrière dans une direction vers un centre de la surface avant,
le boîtier de diffuseur (210) comprenant une portion périphérique avant (236) entourant la surface ouverte,
**caractérisé en ce que** la portion périphérique avant (236) comprend une pluralité de premières portions (237) et une pluralité de secondes portions (238) disposées au moins partiellement en arrière des premières portions (237),
une longueur s'étendant vers l'avant d'au moins une partie de la première portion (237) et de la seconde portion (238) étant modifiée le long de la périphérie de la surface ouverte, et
la pluralité des premières portions (237) et la pluralité des secondes portions (238) étant disposées alternativement l'une par rapport à l'autre le long d'une périphérie de la surface ouverte et s'étendant le long de la périphérie de la surface ouverte, tout en ayant chacune des courbures lorsqu'elles sont vues dans une direction latérale.

9. Sèche-cheveux selon la revendication 8, dans lequel une distance depuis une extrémité avant de chacune des premières portions (237) et des secondes portions (238) jusqu'à la face arrière (212) varie le long d'au moins une zone partielle de l'extrémité avant.

10. Sèche-cheveux selon la revendication 8, dans lequel chaque seconde portion (238) a une forme courbée de manière concave dans une direction arrière, une distance depuis l'extrémité avant de chaque seconde portion (238) jusqu'à la face arrière (212) augmentant dans une direction s'éloignant d'un centre de la seconde portion (238),
chaque première portion (237) ayant une forme courbée de manière convexe dans une direction avant,
une distance depuis l'extrémité avant de chaque première portion (237) jusqu'à la face arrière (212) augmentant dans une direction vers un centre de la première portion (237).
